# FASCICULE DE BREVET EUROPEEN

(11) **EP 3 107 489 B1**
(45) Date de publication et mention de la délivrance du brevet: **05.04.2023**
(21) Numéro de dépôt: 15709267.7
(22) Date de dépôt: 17.02.2015
(51) Int. Cl.: A61F 2/00, D04B 21/12, D04B 1/08

(54) **PROTHESE IMPLANTABLE DESTINEE A REPARER LA PAROI ABDOMINALE DANS LA REGION INGUINALE, ET PROCEDE DE FABRICATION D'UNE TELLE PROTHESE**
IMPLANTIERBARE PROTHESE ZUR REPARATUR DER BAUCHDECKE IM INGUINALBEREICH UND VERFAHREN ZUR HERSTELLUNG SOLCH EINER PROTHESE
IMPLANTABLE PROSTHESIS FOR REPAIRING THE ABDOMINAL WALL IN THE INGUINAL REGION, AND METHOD FOR PRODUCING SUCH A PROSTHESIS

(30) Priorité: 18.02.2014 FR 1451288
(43) Date de publication de la demande: 28.12.2016
(73) Titulaire: Cousin Biotech, 59117 Wervicq Sud (FR)
(72) Inventeur: SOLECKI, Gilles, 59390 Lannoy (FR); VERMET, Guillaume, 59100 Roubaix (FR); NOEL, Stephane, 59496 Hantay (FR)
(74) Mandataire: Cabinet Beau de Loménie
(86) Numéro de dépôt international: PCT/FR2015/050384
(87) Numéro de publication internationale: WO 2015/124861

(56) Documents cités:
- EP-A2- 1 439 796
- WO-A1-96/41588
- WO-A1-2005/094721
- FR-A1- 2 875 396
- US-A- 4 452 245
- US-A1- 2004 029 478
- US-A1- 2007 032 805

## Description

### Arrière-plan de l'invention

La présente invention concerne le domaine technique des prothèses implantables destinées à réparer la paroi abdominale dans la région inguinale par voie laparoscopique, en particulier les prothèses implantables dites de forme anatomique.

On connait des prothèses implantables dites de forme anatomique, c'est-à-dire ayant au moins une partie convexe vue selon la première face de ladite prothèse et correspondant à une région anatomique déterminée disposée à droite ou à gauche de ladite prothèse selon que ladite prothèse soit une prothèse dite « gauche » ou « droite ». Ces prothèses peuvent être tricotées avec un fil mono-filamentaire en polypropylène, et sont généralement destinées à être implantées par deux techniques laparoscopiques.

La première technique laparoscopique dite Totalement Extra-Péritonéale (T.E.P.) consiste à placer la prothèse dans un espace entre le péritoine et la paroi musculaire sans passer par la cavité abdominale et sans dissection du péritoine. Cette technique est particulière puisqu'elle consiste à créer, dans un premier temps, une petite cavité dite extra péritonéale, en décollant en partie le péritoine de la paroi abdominale. Ce décollement est réalisé à l'aide d'un ballonnet placé en position dégonflée entre le péritoine et la paroi abdominale. Le ballonnet est ensuite gonflé, dégonflé et retiré pour laisser en place une petite cavité. La prothèse est ensuite roulée sur elle-même pour être passée au travers de la paroi abdominale par l'intermédiaire d'un trocart, puis disposée dans cette petite cavité créée artificiellement où elle reprend sa forme de façon partielle.

La seconde technique dite TransAbdominale PréPéritonéale (T.A.P.P.) permet de placer la prothèse entre la paroi abdominale et le péritoine en passant par la cavité abdominale en disséquant le péritoine pour le refermer ensuite sur la prothèse. Cette technique consiste à déployer dans un premier temps la prothèse dans la cavité abdominale au contact des viscères et sans utiliser de ballonnet. La prothèse destinée aux deux techniques laparoscopiques possède une partie convexe « droite » ou « gauche » vue selon sa première face qui lui permet de s'adapter à l'anatomie de la région inguinale, droite ou gauche. La prothèse possède également une mémoire partielle de sa forme. En effet, la prothèse reprend une partie de sa forme initiale de façon spontanée après son insertion en position roulée, dans la petite cavité extrapéritonéale artificielle ou la cavité abdominale.

De telles prothèses sont fabriquées par thermoformage d'une surface de base plane tricotée composée de fils monofilamentaires de polypropylène. Cette surface de base plane en polypropylène est placée dans un moule et un contre-moule dont la forme correspond exactement à l'anatomie de la région inguinale. La prothèse est retirée immédiatement après l'étape de thermoformage du moule et du contre-moule, mais ne conserve qu'une partie de la forme impartie par le moule et le contre-moule. Les prothèses tricotées avec des fils en polypropylène ne présentent donc pas une forme identique à la forme de l'anatomie de la zone inguinale après thermoformage. Le travail du chirurgien est donc plus complexe, puisqu'il doit donc compléter la déformation de la prothèse afin qu'elle coopère parfaitement avec la région inguinale à traiter, en particulier au regard de l'orifice herniaire. Cette déformation complémentaire peut être effectuée avec des agrafes et d'autres moyens de suture. Néanmoins, de telles sutures sont au détriment du confort du patient et allongent le temps de l'intervention chirurgicale.

En outre, une prothèse anatomique n'ayant pas une partie convexe vue selon sa première face qui est parfaitement mise en forme a tendance à former des plis au détriment du confort du patient.

Les documents WO 9641588 et FR 2875396 décrivent une telle prothèse « droite » ou « gauche » ayant une partie convexe vue selon sa première face, avec, aux figures 1 et 2 du document WO 9641588, des représentations schématiques des courbures sous forme de lignes perpendiculaires permettant de visualiser ces parties convexes. Ces prothèses sont faciles à mettre en place du fait de leur forme voisine de la forme anatomique de la zone inguinale, mais possèdent cependant une mémoire de leur forme qui est insuffisante pour être placée rapidement en chirurgie. En effet, la cavité extrapéritonéale artificielle entre le péritoine et la paroi abdominale ou la cavité abdominale ont des dimensions relativement faibles. De ce fait, la prothèse ne retrouve qu'une partie de sa forme initiale après avoir été roulée et avoir traversé la paroi abdominale pour être introduite dans la cavité extrapéritonéale artificielle ou la cavité abdominale. La perte d'une partie de cette forme est problématique, en particulier en périphérie de la prothèse qui a tendance à garder la forme roulée que le chirurgien lui a donnée pour la passer à travers la paroi abdominale. Les prothèses perdent aussi une partie de leur concavité ou convexité. Le chirurgien doit l'aider à se déployer à l'aide de deux pinces pour lui permettre de retrouver sa forme initiale. Les contraintes exercées sur la prothèse lors de l'intervention chirurgicale modifient de façon importante sa forme initiale.

Le seul exemple de prothèse décrit en page 5 dans WO 96/41588 est dans un matériau non résorbable. De même dans FR 2.875.396, le seul exemple de prothèse cité en page 3 est dans un tricot indémaillable avec un fil d'environ 150 µm, 100% polypropylène biocompatible.

US 2007/0032805 a pour objet une prothèse en cellulose oxydée comprenant une première face poreuse et une seconde face dense opposées de sorte que le textile comprenant ces deux faces est qualifié de tridimensionnel mais il ne s'agit pas d'une prothèse anatomique comportant au moins une partie convexe de forme anatomique déterminée vue selon au moins une desdites faces. En outre, dans les cinq variantes de réalisation illustrées aux paragraphes 52 à 56 ainsi que dans les exemples 1 à 8, le premier fil résorbable est dans tous les cas un fil multifilamentaire, et de préférence dans une cellulose régénérée subissant un traitement particulier en sorte de la rendre résorbable. US 2007/0032805 ne cherche pas à thermoformer une prothèse mais à prévenir les adhérences postopératoires.

WO 2005/094721 a pour objet un dispositif médical, en particulier une prothèse qui peut être utilisée pour le traitement des hernies. Cette prothèse n'est pas anatomique et comprend une structure dans les 11 exemples de réalisation cités exclusivement constituée de fils monofilamentaires en polypropylène.

Il existe donc un besoin pour une prothèse ayant au moins une partie convexe vue selon une première face, également désignée sous le terme de prothèse anatomique, qui soit apte à adopter précisément la forme anatomique de la région inguinale à traiter après sa mise en forme, et ce de façon stable, même après avoir été enroulée dans un trocart pour son introduction sur la région anatomique à traiter.

### Objet et résumé de l'invention

La présente invention a pour objet, selon un premier aspect, une prothèse implantable destinée à réparer la paroi abdominale dans la région inguinale palliant les problèmes précités, en ce qu'elle comprend une structure de base tricotée ayant des première et seconde faces opposées et comprenant au moins un fil monofilamentaire en polypropylène, des ouvertures ayant au moins une dimension supérieure ou égale à 1 mm, et, vue selon ladite première face, au moins une partie convexe ayant une forme anatomique déterminée, ladite structure de base comprenant avantageusement au moins un fil monofilamentaire en acide polylactique (PLA). De plus, la structure de base tricotée est tricotée à l'aide d'une première barre à passettes supportant au moins un fil monofilamentaire en polypropylène et un fil monofilamentaire en acide polylactique formant un premier fil composite, et d'une deuxième barre à passettes supportant un fil monofilamentaire en acide polylactique, et éventuellement un fil monofilamentaire en polypropylène formant un second fil composite, de préférence les aiguilles de la ou les première et/ou seconde barres sont enfilées un plein au moins un vide.

La prothèse selon l'invention est destinée notamment pour la chirurgie Totalement Extra-Péritonéale (T.E.P) et/ou la chirurgie Trans-Abdominale Pré-Péritonéale (T.A.P.P).

Avantageusement, ladite prothèse est apte à recouvrir ladite forme anatomique après avoir était enroulée sur elle-même.

De manière surprenante, il a été trouvé que les fils monofilamentaires en acide polylactique améliorent nettement la stabilité et la précision de la mise en forme des prothèses comportant au moins une partie convexe vue selon une première face.

Avantageusement, la prothèse droite ou gauche selon l'invention présente, vue selon sa première face, une partie convexe, correspondant à la zone anatomique gauche ou droite de la région inguinale, dont les dimensions sont très proches, voire identiques aux dimensions de ladite zone anatomique, après la mise en forme de ladite prothèse, puis après que cette dernière ait été déployée et déroulée sur le site d'implantation en sortie du trocart. La prothèse selon l'invention possède ainsi une meilleure mémoire de sa forme comparativement à des prothèses de l'état de la technique ne comprenant que des fils monofilamentaires en polypropylène.

L'acide polylactique confère ainsi à la structure de base tricotée -plane en sortie du métier à tricoter- une plus grande aptitude à être thermoformée aux dimensions du moule et du contre-moule comprenant l'empreinte de ladite partie convexe.

Les fils monofilamentaires en polypropylène apportent un renfort mécanique durable à la prothèse selon l'invention, et contribuent en outre au thermoformage de la partie convexe de la prothèse selon l'invention.

Il ne serait pas possible d'utiliser une prothèse en traitement d'une hernie inguinale comprenant exclusivement des fils monofilamentaires en acide polylactique. En effet, il est nécessaire que la prothèse apporte un renfort mécanique sur la durée à la zone anatomique traitée et ne disparaisse pas totalement au bout de quelques semaines du fait de la dégradation de l'acide polylactique. La prothèse selon l'invention doit ainsi apporter un renfort mécanique durable et précis du fait de sa partie convexe afin d'éviter les risques de récidive de la hernie.

Avantageusement, la prothèse selon l'invention comporte des ouvertures et est tricotée, ce qui améliore sa déformabilité et donc ses propriétés de mise en forme lors du thermoformage.

La prothèse selon l'invention, comportant au moins une partie convexe avant implantation correspondant précisément à la zone anatomique à traiter, évite ainsi l'emploi d'agrafes ou de moyens de suture utilisés auparavant dans l'état de la technique pour la mise en forme ou la correction de la mise en forme de ladite prothèse, en particulier au regard de l'orifice herniaire à renforcer. Cette disposition facilite donc le travail du chirurgien et améliore le confort du patient. La mise en forme étant très précise, cela limite également la formation éventuelle de plis par la prothèse.

De préférence, ladite partie convexe vue selon ladite première face a une hauteur déterminée (H) supérieure à zéro et mesurée selon une droite perpendiculaire au plan P dans lequel repose la structure de base. Ladite hauteur déterminée (H) est supérieure ou égale à 5 mm, encore de préférence supérieure ou égale à 10 mm, encore de préférence supérieure ou égale à 20 mm.

L'acide polylactique ou le polylactide (PLA) est un polymère biodégrable et biocompatible. Le PLA utilisé dans le cadre de la présente invention peut être produit par différentes méthodes de production telle que la polymérisation par ouverture de cycle à partir de lactide ou la polymérisation par condensation directe à partir d'acide lactique.

On entend par polymère biodégradable, tout polymère qui, une fois implanté dans le corps, va disparaître au bout d'un temps déterminé. Dans le cadre de la présente invention, le ou les fils monofilamentaires en PLA auront disparu au terme de vingt-quatre mois, de préférence au terme de douze mois, et encore de préférence au terme de six mois

Plus particulièrement, l'acide polylactique selon l'invention est un polyester aliphatique.

Au sens de la présente invention, on entend par x est compris dans l'intervalle [y ; z] que x est supérieur ou égal à y et inférieur ou égal à z.

Avantageusement, la prothèse selon l'invention est semi-résorbable, c'est-à-dire que seulement une partie de la prothèse reste dans le corps de façon durable après implantation. L'acide polylactique se dégrade donc totalement au terme de plusieurs mois, il ne reste alors que le polypropylène qui subsiste de manière durable dans le corps. Cette disposition améliore le confort du patient.

Le ou les fils monofilamentaire(s) en polypropylène peut ou peuvent être un homopolymère de propylène ou un copolymère de propylène et d'autres alpha-oléfines. Des exemples d'alpha-oléfines qui peuvent être copolymérisés comprennent les alpha-oléfines de 2 à 20 atomes de carbone, tel que l'éthylène, le 1-butène, le 1-pentène, le 1-hexène, le 1-octène, le 1-decène, le 3-méthyl-1-butène, le 3-méthyl-1-pentène, le 3-éthyl-1-pentène, le 4-méthyl-1-pentène et le 4-méthyl-1-hexène. L'éthylène et le 1-butène sont préférés, en particulier l'éthylène. De tels alpha-oléfines peuvent être polymérisés seuls ou en combinaison à deux ou plus.

De préférence, le ou les fils monofilamentaire(s) sont dans un homopolymère de polypropylène, de préférence isotactique.

Avantageusement, la structure de base comprend un fil monofilamentaire en polypropylène et un fil monofilamentaire en acide polylactique adjacents et formant les mêmes mailles ensemble puisque supportés par la même barre à passettes.

Cette disposition renforce les propriétés de thermoformage de la prothèse selon l'invention puisqu'un fil monofilamentaire en PP est suivi d'un fil monofilamentaire en PLA.

De plus, cette disposition assure que lors de la dégradation du ou des fils en PLA, la structure de base maintient une certaine cohésion et donc assure sa fonction première de renfort mécanique.

L'enfilage de la première barre à passettes et/ou de la seconde barre à passettes permet de ménager des ouvertures dans la structure de base.

De préférence, la structure de base tricotée est tricotée selon une jauge de 15 à 25 aiguilles par pouce (un pouce étant équivalent à 25,4 mm), encore de préférence entre 17 et 22 aiguilles par pouce.

Dans une variante, le premier fil composite, et éventuellement le second fil composite, est ou sont retordu(s), selon un nombre de tours/mètre compris entre 1 tour/mètre et 120 tours/mètre.

Cette disposition permet de faciliter le tricotage, notamment par une meilleure gestion des tensions qui s'exercent sur le ou les fils composite(s).

Dans une variante, ledit au moins un fil monofilamentaire en acide polylactique (PLA) a une Longueur Thermoformée des Spires (L.T.S) comprise dans l'intervalle [3 mm ; 7 mm].

Dans une variante, ledit au moins un fil monofilamentaire en acide polylactique a une Longueur Résiduelle de Spires (L.R.S.) comprise dans l'intervalle [15 mm ; 42 mm].

Il a été déterminé que le ou les fils monofilamentaire(s) en acide polylactique donnant des résultats nettement améliorés en terme de stabilité et de précision de la forme de la prothèse selon l'invention, a ou ont une LTS, éventuellement combinée avec une LRS, tombant dans le ou les intervalle(s) déterminé(s) décrit(s) ci-dessus.

Il est difficile d'évaluer le comportement d'un fil à un autre vis-à-vis du thermoformage ou encore sa mémoire de forme lors de déploiement en sortie du trocart. En effet, ces comportements dépendent de nombreux paramètres dont le taux de cristallinité du polymère, les paramètres de fabrication du fil dont le taux d'étirage ou encore le taux de cisaillement lors de l'extrusion-filage. Les valeurs de LTS et LRS selon l'invention permettent d'apprécier ces comportements spécifiques et recherchés pour la prothèse que ce soit lors du thermoformage ou lors du déploiement de la prothèse sur le site d'implantation.

La méthode de mesure des valeurs LTS et LRS est décrite ci-après en détail dans la partie descriptive.
Dans une variante, ledit au moins un fil monofilamentaire en acide polylactique comprend au plus 10 % en poids de son poids d'unités de forme D, de préférence au plus 5 % en poids de son poids d'unités de forme D, encore de préférence la quantité en poids de son poids d'unités de forme D est supérieure à 0% et inférieure ou égale à 3%, et encore de préférence la quantité en poids de son poids d'unités de forme D est comprise entre 0,5% et 2%. On entend par unité de forme D, l'isomère de forme D.

Dans une variante, la structure de base tricotée est à mailles jetées, et ledit au moins un fil monofilamentaire en polypropylène et ledit au moins un fil monofilamentaire en acide polylactique forment des mailles tricots s'étendant sur au moins trois colonnes de mailles et forment une maille ouverte toute les une maille fermée à toutes les quatre mailles fermées.

Cette disposition améliore encore la déformabilité de la prothèse et facilite ainsi sa mise en forme par thermoformage.

Cette disposition permet avantageusement de former des ouvertures ayant une forme allongée ou ovoïde favorisant la déformabilité et donc la conformabilité de la prothèse lors du thermoformage.

Dans une variante, ledit au moins un fil monofilamentaire en polypropylène et/ou ledit au moins un fil monofilamentaire en acide polylactique a ou ont un diamètre compris dans l'intervalle [0,08 mm ; 0,30 mm], de préférence dans l'intervalle [0,10 mm ; 0,20 mm].

Ces intervalles de diamètre permettent d'obtenir une prothèse ayant des propriétés de thermoformage améliorées. Le ou les fils ne doivent pas en effet présenter une section transversale trop importante afin de pouvoir être thermoformables et non abrasifs pour le patient, ni une section trop faible au risque de ne plus être thermoformables et ne plus assurer un renfort mécanique convenable.

Dans une variante, les ouvertures de la structure de base ont une forme allongée, ovoïde ou elliptique dont la longueur du grand axe (mm) est comprise entre deux et quatre fois la longueur du petit axe (mm).

La taille des ouvertures de la structure de base est importante afin qu'elle soit thermoformable.

On entend par au moins une dimension dans cette variante précise, la longueur du grand axe ou la longueur du petit axe.

Cette configuration contribue à faciliter la déformation, pendant le processus de thermoformage, de la structure de base plane tricotée, dans le sens du petit axe de l'ouverture ou orifice. La structure de base a de préférence une capacité de s'allonger entre 10 % et 200 % dans la direction du petit axe, ce qui permet, grâce à cette caractéristique complémentaire, une plus grande conformation de la structure de base plane tricotée aux différentes empreintes que peut comprendre le moule et le contre-moule.

Dans une variante, les longueurs dudit grand axe et dudit petit axe sont comprises dans l'intervalle [1 mm ; 5 mm].

La présente invention a également pour objet, selon un second aspect, un procédé de fabrication d'une prothèse implantable selon l'une quelconque des variantes de réalisation précédentes, comprenant avantageusement les étapes suivantes :
a. une étape de tricotage d'une structure de base plane ayant des première et seconde faces opposées, à mailles jetées, à partir d'un fil monofilamentaire en polypropylène (PP) et d'un fil monofilamentaire en acide polylactique (PLA), ladite structure ayant des ouvertures dont au moins une dimension est supérieure ou égale à 1 mm, la structure de base tricotée est tricotée à l'aide d'une première barre à passettes supportant au moins un fil monofilamentaire en polypropylène et un fil monofilamentaire en acide polylactique formant un premier fil composite, et d'une deuxième barre à passettes supportant un fil monofilamentaire en acide polylactique, et éventuellement un fil monofilamentaire en polypropylène formant un second fil composite;
b. une étape de thermoformage de ladite structure de base plane au cours de laquelle au moins une partie de ladite structure est soumise à une déformation mécanique sous l'application d'une température déterminée et pendant une période déterminée pour conférer, vue selon ladite première face de la structure de base, une forme convexe à ladite partie correspondant à une forme anatomique déterminée.

De préférence, lors de l'étape de thermoformage, la structure de base plane tricotée est disposée dans un moule comportant une empreinte en creux destinée à former ladite partie convexe de forme anatomique déterminée. De préférence, ladite structure de base tricotée est mise en tension sur ledit moule, par exemple en solidarisant ladite structure de base en différents points audit moule, notamment à l'aide de pointes. Puis le contre-moule comportant une empreinte complémentaire de celle du moule, est disposée sur le moule. Le moule et le contre-moule sont portés à une température comprise entre 135°C et 150°C. La structure de base plane est comprimée entre le moule et le contre-moule pendant une durée comprise entre 30 secondes et 30 minutes, de préférence entre 1 minute et 15 minutes, puis le moule et le contre-moule sont refroidis à température comprise entre 20°C et 25°C pendant une durée de 1 minute à 30 minutes, de préférence 15 minutes, tout en maintenant comprimée ladite structure de base tricotée. Ladite structure de base est ensuite ôtée desdits moule et contre-moule et présente alors, vue selon ladite première face, une partie convexe ayant une forme anatomique déterminée correspondant à l'empreinte convexe dudit moule.

De préférence, la structure de base tricotée, ayant une partie convexe, présente les caractéristiques techniques décrites ci-dessus en référence à la prothèse selon un premier aspect, seule ou en combinaison.

Dans une variante, ledit procédé comprend une étape de sélection dudit fil monofilamentaire en acide polylactique (PLA), préliminaire à l'étape a), consistant à sélectionner un fil monofilamentaire ayant une Longueur Thermoformée des Spires (L.T.S) comprise dans l'intervalle [3 mm ; 7 mm], et de préférence une Longueur Résiduelle de Spires (L.R.S.) comprise dans l'intervalle [15 mm ; 42 mm].

### Description détaillée des dessins

La présente invention sera mieux comprise à la lecture de la description d' exemples de réalisation de prothèses selon l'invention, cités à titres non limitatifs, et illustrés par les figures suivantes, annexées à la présente et dans lesquelles :
- les figures 1 et 2 sont des représentations schématiques de différentes étapes de la méthode de mesure des valeurs LTS et LRS selon l'invention ;
- la figure 3 est une représentation schématique d'un premier exemple de schéma de mailles utilisé dans le tricotage d'un premier exemple de prothèse selon l'invention ;
- la figure 4 est une représentation schématique du premier exemple de prothèse selon l'invention ayant une partie convexe vue selon sa première face ;
- la figure 5 est une représentation schématique et agrandie de la structure de base tricotée du premier exemple de prothèse représentée à la figure 4 ;
- la figure 6 représente la hauteur de la partie convexe du premier exemple de prothèse selon l'invention vue selon sa première face ;
- la figure 7 est une représentation schématique d'un second exemple de schéma de mailles utilisé pour le tricotage d'un second exemple de prothèse selon l'invention.

### Description détaillée de la méthode de mesure des valeurs LRS et LTS selon l'invention d'un fil monofilamentaire ayant un diamètre compris entre 0,10 mm et 0,20 mm.

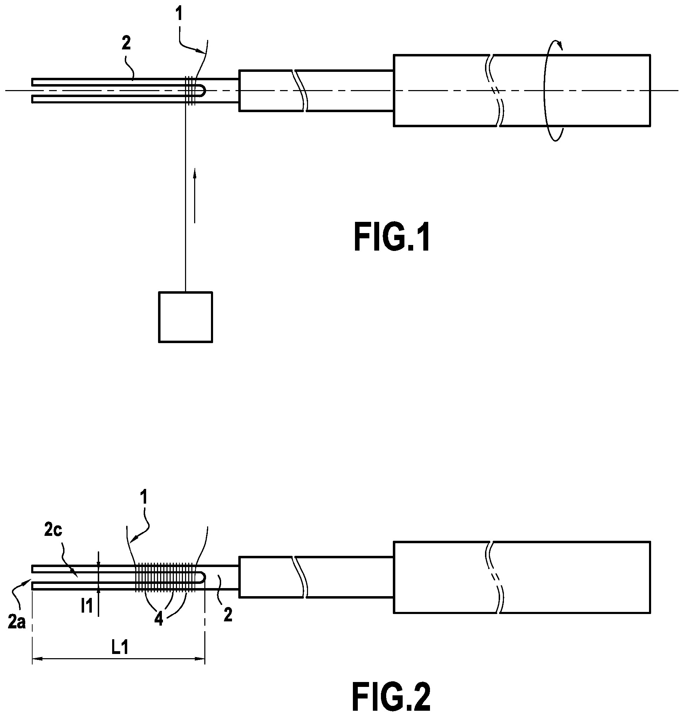

a. Conditionner le fil monofilamentaire à 23°C et sous une humidité relative de 40 % pendant une durée de 48 heures, tel que le fil 1 représenté aux figures 1 et 2.
b. Enrouler ensuite le fil monofilamentaire, tel que le fil 1 représenté à la figure 2, sur un mandrin 2 en inox 316L de 30 cm de longueur, de diamètre 2,5 mm et comportant à son extrémité 2a une fente 2c selon son axe de largeur I1 de 1 mm et de longueur L1 de 8 mm. Le fil monofilamentaire, tel que le fil 1, est introduit dans la fente 2c, enroulé sur 20 tours à une vitesse de 1 tour/seconde, sous une force de 2 N, tel que représenté à la figure 1, et réintroduit dans la fente 2c. Les spires 4 sont enroulées de façon jointive à 23°C et sous une humidité relative de 40 %. Les deux extrémités du fil monofilamentaire, tel que le fil 1, sortant du même côté de la fente 2c sont ensuite nouées. La Longueur Initiale des Spires (L.I.S.) est mesurée selon l'axe des spires et sur une distance comprise entre le début de la première spire et la fin de la dernière spire.
c. Traiter thermiquement le fil monofilamentaire sur le mandrin 2 à 135°C pendant 15 mn sous une humidité relative de 40 %.
d. Laisser refroidir le fil monofilamentaire sur le mandrin 2 pendant 15 mn à 23°C et sous une humidité relative de 40 %.
e. Couper les brins du fil monofilamentaire en amont des noeuds puis retirer les spires du mandrin 2 en les poussant doucement et mesurer immédiatement la Longueur Thermoformée des Spires (L.T.S.) à 23°C sous une humidité relative de 40 %.
   Cette longueur est mesurée selon l'axe passant sensiblement par le centre des spires et entre le début de la première spire et la fin de la dernière spire. Avantageusement, la valeur de L.T.S permet de caractériser la capacité du fil monofilamentaire à être thermoformé aux dimensions de l'empreinte du moule. Plus la valeur de L.T.S est faible, et de préférence proche de la valeur de L.I.S dont la mesure est décrite ci-dessus, plus la structure de base tricotée comprenant le fil monofilamentaire satisfaisant à la valeur de L.T.S déterminée aura une aptitude à être thermoformée aux dimensions de l'empreinte moule, et de ce fait, aux dimensions de la zone inguinale.
f. Etirer les spires à une longueur d'étirage Lo de 150 mm sous une vitesse de 50 mm/seconde, par exemple à l'aide d'un banc de traction, tels que ceux commercialisés sous la marque Instron. L'étirage des spires est réalisé à 23°C sous une humidité relative de 40 %, selon l'axe passant sensiblement par le centre des spires, et entre les points compris entre le début de la première spire et la fin de la dernière spire. Cette longueur d'étirage Lo est ensuite maintenue pendant 30 secondes à 23°C et sous une humidité relative de 40 %.
g. Relâcher l'effort d'étirage et mesurer la Longueur Résiduelle des Spires (L.R.S.) entre le début de la première spire et la fin de la dernière spire après un temps de relaxation de 10 mn. La mesure de la L.R.S est réalisée à 23°C sous une humidité relative de 40 %.
   Cette étape simule les contraintes exercées sur le fil monofilamentaire dans la prothèse lors de son placement par le chirurgien sur le site d'implantation. Plus la valeur de L.R.S est faible, et de préférence proche de la valeur de L.T.S. décrite ci-dessus, plus la mémoire de forme de la prothèse sera importante.

Cette méthode de mesure des valeurs de L.R.S et de L.T.S permet de caractériser les fils monofilamentaires et de choisir ensuite ceux qui seront les plus adaptés au thermoformage. Un des buts de l'invention est de réduire les écarts de forme importants entre l'empreinte du moule de fabrication, qui a la forme anatomique de la zone inguinale, et la forme de la partie convexe de la prothèse.

Il a ainsi été observé que plus la valeur L.T.S. est faible, plus le fil monofilamentaire a une capacité à adopter la forme exacte de l'empreinte du moule.

Plus la valeur L.R.S est faible plus la prothèse aura la capacité à retrouver sa forme, après avoir subi une contrainte de durée courte, tel que l'enroulage de ladite prothèse par le chirurgien sur elle-même et son introduction dans la cavité extrapéritonéale ou abdominale.

Le tableau 1 ci-après reprend les valeurs de LTS et LRS mesurées selon la méthode de mesure décrite ci-dessus sur différents fils numérotés de 1 à 7 en acide polylactique.

**Tableau 1**

| Fils Monofilamentaires d'acide polylactique | | | | |
|---|---|---|---|---|
| N° du fil et Taux de cristallinité du polymèrecomposant ledit fil monofilamentaire | Pourcentage en poids d'isomère de forme D dans l'acide polylactique | Diamètre du fil monofilamentaire (mm) | L.T.S (mm) | L.R.S (mm) |
| Fil n°1 | 0.5 | 0.15 mm | 3.5 mm | 15 mm |
| 50-60 % | | | | |
| Fil n°2 | 1.0 | 0.15 mm | 4.5 mm | 29 mm |
| 40-50 % | | | | |
| Fil n°3 | 1.0 | 0.15 mm | 4.5 mm | 31 mm |
| 40-50 % | | | | |
| Fil n°4 | 1.0 | 0.15 mm | 5.0 mm | 36 mm |
| 40-50 % | | | | |
| Fil n°5 | 1.0 | 0.15 mm | 5.0 mm | 30 mm |
| 40-50 % | | | | |
| Fil n°6 | 2.0 | 0.15 mm | 7.0 mm | 42 mm |
| 25-35 % | | | | |
| Fil n°7 | 1.0 | 0.20 mm | 5.5 mm | 39 mm |
| 40-50 % | | | | |
| 40-50 % | | | | |

Le tableau 2 ci-après reprend les valeurs de LTS et LRS mesurées selon la méthode de mesure décrite ci-dessus sur différents fils numérotés 8 à 12 en polypropylène, en particulier les fils testés n°8 à 12 sont dans un homopolymère de polypropylène isotactique.

**Tableau 2**

| Fils monofilamentaires de polypropylène | | | |
|---|---|---|---|
| N° du fil et taux de cristallinité du polymère composant le fil monofilamentaire | Diamètre du monofilament (mm) | L.T.S (mm) | L.R.S (mm) |
| Fil n°8 | 0.15 mm | 13.0 mm | 43 mm |
| 66 - 70 % | | | |
| Fil n°9 | 0.15 mm | 10.0 mm | 45 mm |
| 67 - 71 % | | | |
| Fil n°10 | 0.15 mm | 9.0 mm | 50 mm |
| 70 - 74 % | | | |
| Fil n°11 | 0.20 mm | 13.0 mm | 50 mm |
| 69 - 73 % | | | |
| Fil n°12 | 0.20 mm | 12.0 mm | 49 mm |
| 68 - 72 % | | | |

Le tableau 3 ci-après reprend les valeurs de LTS et LRS mesurées selon la méthode de mesure décrite ci-dessus sur différents fils numérotés 13 à 16 dans différents polymères.

**Tableau 3**

| Fils monofilamentaires | | | | |
|---|---|---|---|---|
| N° fil et nature | Taux de cristallinité du polymère composant le fil monofilamentaire | Diamètre du fil (mm) | L.T.S (mm) | L.R.S (mm) |
| Fil n°13 | 42 - 45 % | 0.15 mm | 12.0 mm | 26 mm |
| Polyéthylène téréphtalate | | | | |
| Fil n°14 | 30 - 35 % | 0.15 mm | 32.0 mm | 63 mm |
| Polyéther éther Ketone | | | | |
| Fil n°15 | 40 - 45 % | 0.16 mm | 19.0 mm | 42 mm |
| Polyamide 6 | | | | |
| Fil n°16 | 42 - 47 % | 0.10 mm | 10.0 mm | 31 mm |
| Polyfluorure de vinylidène | | | | |

### Commentaires des tableaux 1 à 3

Dans le cas des fils monofilamentaires de polypropylène n° 8 à 12 dans le tableau 2, la L.T.S est comprise entre 9 mm et 13 mm. Dans le cas des fils monofilamentaires de polylactide (PLA) n° 1 à 7, la L.T.S. est comprise entre 3,5 mm et 7,0 mm. Les fils monofilamentaires de polylactide présentent donc une plus grande capacité à adopter les dimensions de l'empreinte d'un moule comparativement aux fils monofilamentaires en polypropylène.

Dans le cas des fils monofilamentaires en polypropylène n° 8 à 12, la L.R.S est comprise entre 43 mm et 50 mm. Dans le cas des fils monofilamentaires de polylactide n° 1 à 7, la L.T.S. est comprise entre 15 mm et 42 mm. Les fils monofilamentaires de polylactide ont donc plus de capacité à retrouver leur forme après avoir été contraints pendant une durée courte par rapport aux fils monofilamentaires de polypropylène.

On remarque également que parmi les fils monofilamentaires cités au tableau 3, la valeur de L.T.S la plus faible de 10 mm est obtenue pour un fil en polyfluorure de vinylidène, cette valeur étant supérieure à la valeur de L.T.S de 7 mm la plus élevée mesurée sur un fil en PLA. Les fils en PLA ont ainsi un comportement au thermoformage nettement amélioré comparativement aux fils dans différents polymères testés.

### Description détaillée d'exemples de réalisation de prothèses selon l'invention

### Exemple 1

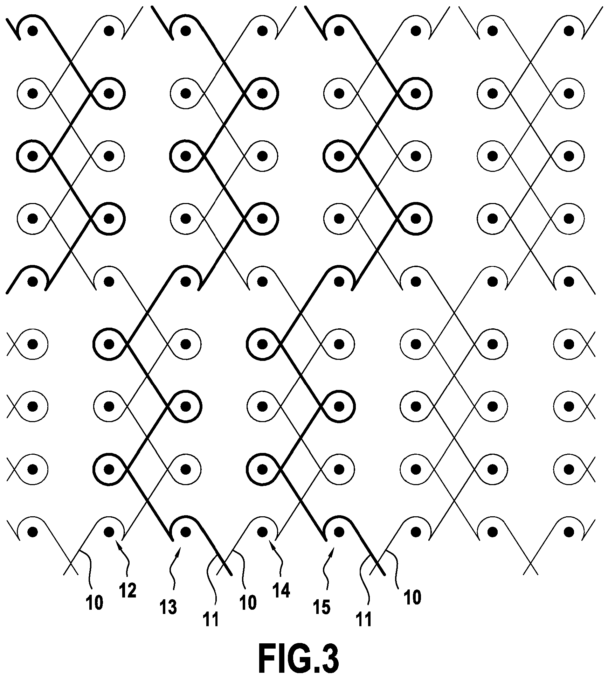

La structure de base comprend des mailles jetées, en particulier des mailles tricotées s'étendant sur trois colonnes de mailles. La structure de base est tricotée avec un premier fil composite 10 représenté sur le schéma de mailles à la figure 3 et comprenant un fil monofilamentaire en polypropylène de diamètre 0,15 mm et un fil monofilamentaire de polylactide de diamètre 0,15 mm, ledit premier fil composite n'est pas retordu. La structure de base comprend également un second fil composite 11 identique au premier fil composite 10. Les premier 10 et second 11 fils composites s'étendent sur trois colonnes de mailles, par exemples les colonnes de mailles 12, 13 et 14. Une colonne de mailles correspond sur le schéma de mailles à une ligne de points noirs horizontale représentant les aiguilles de tricotage. Le premier fil 10 et le second fil 11 forment des mailles ouvertes 15 à intervalles réguliers, en particulier toutes les trois mailles tricotées. Les premiers 10 et second 11 fils composites sont ourdis sur des ensouples avant le tricotage. La structure de base est réalisée sur un métier Rachel possédant de préférence 18 aiguilles au pouce (1 pouce = 25,4 mm). La structure de base est réalisée à l'aide de deux barres à passettes avec un enfilage un plein - un vide sur chaque barre. Les première et seconde barres supportent respectivement le premier fil composite et le second fil composite. L'amure de tricotage est la suivante :
- mouvement première barre: 2-4 / 4-6 / 4-2 / 4-6 / 4-2 / 2-0 / 2-4 / 2-0 //
- mouvement seconde barre : 6-4 / 4-2 / 4-6 / 4-2 / 4-6 / 6-8 / 6-4 / 6-8 //

Le fil monofilamentaire en polypropylène utilisé pour les fils composites 10 et 11 correspond dans cet exemple précis, au fil numéroté 8 au tableau 2.

Le fil monofilamentaire en acide polylactique utilisé pour les fils composites 10 et 11 correspond dans cet exemple précis, au fil numéroté 1 au tableau 1.

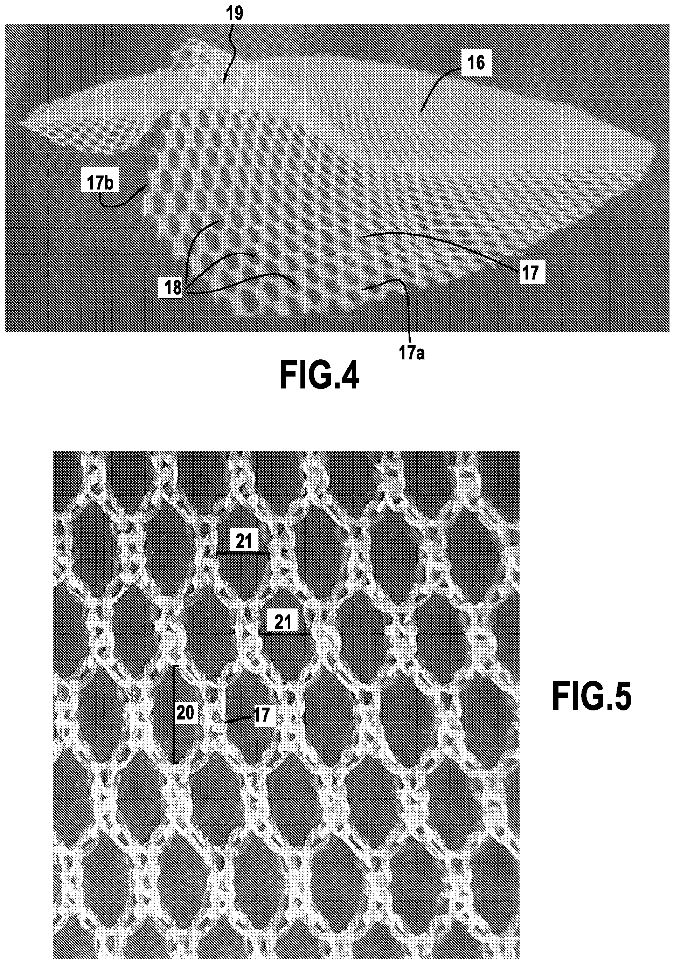

La figure 4 représente schématiquement le premier exemple de prothèse 16 selon l'invention obtenu selon l'exemple 1 et comprenant une structure de base 17, ayant des première 17a et seconde 17b faces opposés, et des ouvertures 18 dont au moins une dimension est supérieure à 1 mm, ainsi qu'une partie convexe 19, vue selon la première face 17a, mise en forme selon une forme anatomique déterminée.

La figure 5 représente en détails la structure de base 17 de la prothèse 16, en particulier les orifices ou ouvertures 18 de forme elliptique obtenus. Dans cet exemple précis, le grand axe 20 desdites ouvertures 18 a une longueur de 4,5 mm et le petit axe 21 desdites ouvertures 18 a une longueur de 1,5 mm. Cette structure de base tricotée 17 a pour avantage qu'elle est déformable et ainsi présente des propriétés améliorées vis-à-vis du thermoformage.

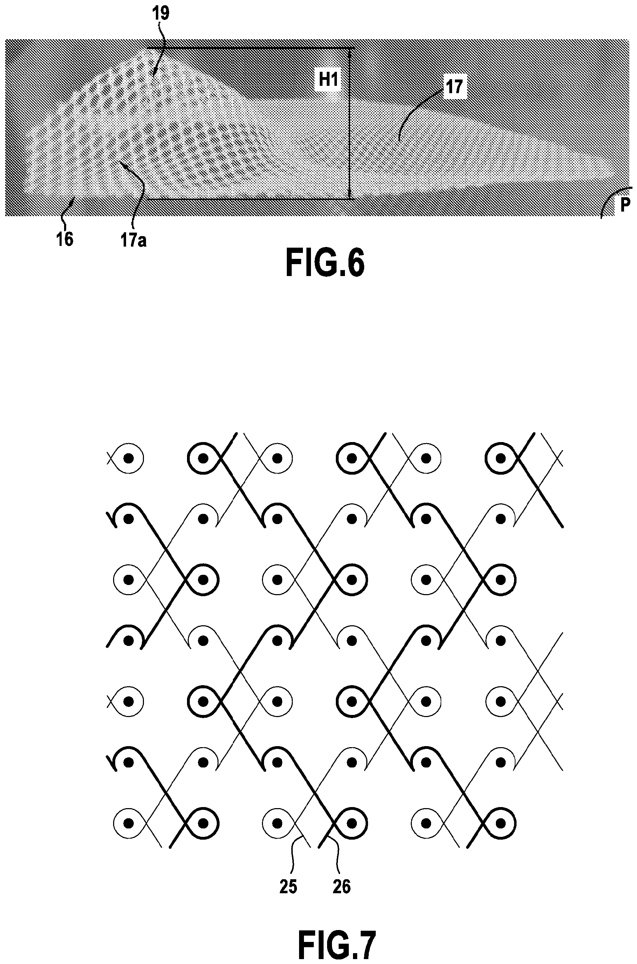

La figure 6 représente de manière schématique la prothèse 16 disposée sur un plan P. La hauteur H1 de la partie convexe 19, mesurée selon une droite perpendiculaire au plan P, est de l'ordre de 28 mm, cette hauteur H1 est mesurée sur la prothèse après son thermoformage. La hauteur correspondante sur l'empreinte du moule utilisé pour le thermoformage de la structure de base 17 est également de 28 mm. Comparativement, cette hauteur de la partie convexe 19 vue selon la première face 17a a été mesurée dans les mêmes conditions pour une prothèse comprenant exclusivement des fils monofilamentaires en polypropylène de diamètre 0,15 mm, et la hauteur obtenue est de 22 mm. On observe ainsi que l'utilisation de fils monofilamentaires en acide polylactique, en particulier ayant des valeurs de L.T.S comprise entre 3 mm et 7 mm, permet d'améliorer très significativement la thermoformabilité de la prothèse 16 selon l'invention à la forme anatomique désirée, en particulier correspondant à l'empreinte précise du moule utilisé.

### Exemple 2

La structure de base est tricotée à mailles jetées avec un premier fil composite comprenant un fil monofilamentaire de polypropylène de diamètre 0,10 mm et un fil monofilamentaire de polylactide de diamètre 0,15 mm, et un second fil composite identique au premier fil composite. Les premier et second fils composites ont été, préalablement à l'étape de tricotage de ladite structure de base plane, retordus à 80 tours par mètre puis bobinés sur un tube perforé, et fixés à une température de 120°C pendant 60 minutes. Les premier et second fils composites sur tube sont ensuite ourdis sur des ensouples pour être ensuite tricotés sur un métier Rachel possédant 18 aiguilles au pouce (1 pouce = 25,4 mm). La structure de base est réalisée à l'aide de deux barres à passettes avec un enfilage un plein - un vide sur chaque barre. Les première et seconde barres supportent respectivement le premier fil composite et le second fil composite. L'amure de tricotage est la suivante :
- mouvement première barre: 2-4 / 4-6 / 4-2 / 4-6 / 4-2 / 2-0 / 2-4 / 2-0 //
- mouvement seconde barre : 6-4 / 4-2 / 4-6 / 4-2 / 4-6 / 6-8 / 6-4 / 6-8 //

### Exemple 3

La structure de base est tricotée à mailles jetées avec un premier fil composite comprenant un fil monofilamentaire de polypropylène de diamètre 0,10 mm et un fil monofilamentaire de polylactide de diamètre 0,20 mm, et un second fil composite identique au premier fil composite. Les premier et second fils composites ont été, préalablement à l'étape de tricotage de ladite structure de base plane, retordus à 100 tours par mètre puis bobinés sur un tube perforé, et fixés à une température de 120°C pendant 60 minutes. Les premier et second fils composites sur tube sont ensuite ourdis sur des ensouples pour être ensuite tricotés sur un métier Rachel possédant 20 aiguilles au pouce (1 pouce = 25,4 mm). La structure de base est réalisée à l'aide de deux barres à passettes avec un enfilage un plein - un vide sur chaque barre. Les première et seconde barres supportent respectivement le premier fil composite et le second fil composite. L'amure de tricotage est la suivante :
- mouvement première barre : 4-2 / 2-0 / 2-4 / 4-6 //
- mouvement seconde barre : 4-6 / 6-8 / 6-4 / 4-2 //

Le schéma de mailles utilisé est représenté à la figure 7. Les premier 25 et second 26 fils composites forment des mailles tricots sur trois colonnes de mailles, et en particulier forment une maille ouverte toute les une maille fermée.

### Exemple 4

La structure de base est tricotée à mailles jetées avec un premier fil composite comprenant un fil monofilamentaire de polypropylène de diamètre 0,20 mm et un fil monofilamentaire de polylactide de diamètre 0,15 mm, et un second fil composite identique au premier fil composite. Les premier et second fils composites ont été, préalablement à l'étape de tricotage de ladite structure de base plane, retordus à 100 tours par mètre puis bobinés sur un tube perforé, et fixés à une température de 120°C pendant 60 minutes. Les premier et second fils composites sur tube sont ensuite ourdis sur des ensouples pour être ensuite tricotés sur un métier Rachel possédant 20 aiguilles au pouce (1 pouce = 25,4 mm). La structure de base est réalisée à l'aide de deux barres à passettes avec un enfilage un plein - un vide sur chaque barre. Les première et seconde barres supportent respectivement le premier fil composite et le second fil composite. L'amure de tricotage est la suivante :
- mouvement première barre : 4-2 / 2-0 / 2-4 / 4-6 //
- mouvement seconde barre : 4-6 / 6-8 / 6-4 / 4-2 //

### Exemple 5

La structure de base est tricotée à mailles jetées avec un premier fil composite comprenant un fil monofilamentaire de polypropylène de diamètre 0,20 mm et un fil monofilamentaire de polylactide de diamètre 0,20 mm, et un second fil composite identique au premier fil composite. Les premier et second fils composites ont été, préalablement à l'étape de tricotage de ladite structure de base plane, retordus à 80 tours par mètre puis bobinés sur un tube perforé, et fixés à une température de 120°C pendant 30 minutes. Les premier et second fils composites sur tube sont ensuite ourdis sur des ensouples pour être ensuite tricotés sur un métier Rachel possédant 20 aiguilles au pouce (1 pouce = 25,4 mm). La structure de base est réalisée à l'aide de deux barres à passettes avec un enfilage un plein - un vide sur chaque barre. Les première et seconde barres supportent respectivement le premier fil composite et le second fil composite. L'amure de tricotage est la suivante :
- mouvement première barre : 4-2 / 2-0 / 2-4 / 4-6 //
- mouvement seconde barre : 4-6 / 6-8 / 6-4 / 4-2 //

### Exemple 6

La structure de base est tricotée à mailles jetées avec un premier fil composite comprenant un fil monofilamentaire de polypropylène de diamètre 0,20 mm et un fil monofilamentaire de polylactide de diamètre 0,20 mm, et un second fil composite identique au premier fil composite. Les premier et second fils composites ont été, préalablement à l'étape de tricotage de ladite structure de base plane, retordus à 80 tours par mètre puis bobinés sur un tube perforé, et fixés à une température de 120°C pendant 30 minutes. Les premier et second fils composites sur tube sont ensuite ourdis sur des ensouples pour être ensuite tricotés sur un métier Rachel possédant 20 aiguilles au pouce (1 pouce = 25,4 mm). La structure de base est réalisée à l'aide de deux barres à passettes avec un enfilage un plein - un vide sur chaque barre. Les première et seconde barres supportent respectivement le premier fil composite et le second fil composite. L'amure de tricotage est la suivante :
- mouvement première barre : 4-2 / 2-0 / 2-4 / 4-6 //
- mouvement seconde barre : 4-6 / 6-8 / 6-4 / 4-2 //

### Exemple 7

La structure de base est tricotée à mailles jetées avec un premier fil composite 25 comprenant un fil monofilamentaire de polypropylène de diamètre 0,20 mm et un fil monofilamentaire de polylactide de diamètre 0,20 mm, et un second fil composite 26 identique au premier fil composite. Les premier et second fils composites ont été, préalablement à l'étape de tricotage de ladite structure de base plane, retordus à 80 tours par mètre puis bobinés sur un tube perforé, et fixés à une température de 120°C pendant 60 minutes. Les premier et second fils composites sur tube sont ensuite ourdis sur des ensouples pour être ensuite tricotés sur un métier Rachel possédant 20 aiguilles au pouce (1 pouce = 25,4 mm). La structure de base est réalisée à l'aide de deux barres à passettes avec un enfilage deux pleins - deux vides sur chaque barre. Les première et seconde barres supportent respectivement le premier fil composite et le second fil composite. L'amure de tricotage est la suivante :
- mouvement première barre : 2-0 / 2-4 / 2-0 / 2-4 / 2-0 / 2-4 / 4-6 / 4-2 / 4-6 / 4-2 / 4-6 / 4-2 //
- mouvement seconde barre : 4-6 / 4-2 / 4-6 / 4-2 / 4-6 / 4-2 / 2-0 / 2-4 / 2-0 / 2-4 / 2-0 / 2-4 //

Dans les exemples 2 à 7, les fils monofilamentaires en acide polylactique ont de préférence une valeur de LTS comprise dans l'intervalle [3mm ; 7mm]. De préférence, lesdits fils monofilamentaires en acide polylactique ont une valeur de LRS comprise dans l'intervalle [15 mm ; 42 mm].

Le fil monofilamentaire en polypropylène utilisé dans les exemples 2 à 7 est de préférence choisi en sorte de présenter la plus faible valeur de LTS, et éventuellement la plus faible valeur de LRS, parmi les fils répertoriés au tableau 2, en respectant les diamètres requis dans chaque exemple.

## Revendications

1. Prothèse implantable (16) destinée à réparer la paroi abdominale dans la région inguinale, notamment pour la chirurgie Totalement Extra-Péritonéale (T.E.P) et/ou la chirurgie Trans-Abdominale Pré-Péritonéale (T.A.P.P), comprenant une structure de base (17) tricotée comprenant au moins un fil monofilamentaire en polypropylène et ayant des première (17a) et seconde (17b) faces opposées ainsi que des ouvertures (18) ayant au moins une dimension supérieure ou égale à 1 mm, et, vue selon ladite première face (17a), au moins une partie convexe (19) ayant une forme anatomique déterminée, **caractérisée en ce que** la structure de base (17) comprend au moins un fil monofilamentaire en acide polylactique (PLA) et **en ce que** la structure de base (17) tricotée est tricotée à l'aide d'une première barre à passettes supportant au moins un fil monofilamentaire en polypropylène et un fil monofilamentaire en acide polylactique formant un premier fil composite (10,25), et d'une deuxième barre à passettes supportant un fil monofilamentaire en acide polylactique.

2. Prothèse implantable (16) selon la revendication 1, **caractérisée en ce que** la deuxième barre à passettes supporte le fil monofilamentaire en acide polylactique et un fil monofilamentaire en polypropylène formant un second fil composite (11,26).

3. Prothèse implantable (16) selon l'une ou l'autre des revendications 1 et 2, **caractérisée en ce que** ledit au moins un fil monofilamentaire en acide polylactique a une Longueur Thermoformée des Spires (L.T.S) comprise dans l'intervalle [3 mm ; 7 mm].

4. Prothèse implantable (16) selon l'une quelconque des revendications 1 à 3, **caractérisée en ce que** ledit au moins un fil monofilamentaire en acide polylactique a une Longueur Résiduelle de Spires (L.R.S.) comprise dans l'intervalle [15 mm ; 42 mm].

5. Prothèse implantable (16) selon l'une quelconque des revendications 1 à 4, **caractérisée en ce que** ledit au moins un fil monofilamentaire en acide polylactique comprend au plus 10 % en poids de son poids d'unités de forme D, de préférence au plus 5 % en poids de son poids d'unités de forme D.

6. Prothèse implantable (16) selon l'une quelconque des revendications 1 à 5, **caractérisée en ce que** la structure de base (17) tricotée est à mailles jetées, et **en ce que** ledit au moins un fil monofilamentaire en polypropylène et ledit au moins un fil monofilamentaire en acide polylactique forment des mailles tricots s'étendant sur au moins trois colonnes de mailles (12,13,14) et forment une maille ouverte (15) toutes les une maille fermée à toutes les quatre mailles fermées.

7. Prothèse implantable (16) selon l'une quelconque des revendications 1 à 6, **caractérisée en ce que** les aiguilles sur la ou les première et/ou seconde barres est/sont enfilée(s) au moins un plein au moins un vide.

8. Prothèse implantable (16) selon la revendication 1, **caractérisée en ce que** le premier fil composite (10,25) est retordu selon un nombre de tours/mètre compris entre 1 tour/mètre et 120 tours/mètre.

9. Prothèse implantable (16) selon la revendication 2, **caractérisée en ce que** le second fil composite (11,26) est retordu selon un nombre de tours/mètre compris entre 1 tour/mètre et 120 tours/mètre.

10. Prothèse implantable (16) selon l'une quelconque des revendications 1 à 9, **caractérisée en ce que** ledit au moins un fil monofilamentaire en polypropylène et/ou ledit au moins un fil monofilamentaire en acide polylactique a ou ont un diamètre compris dans l'intervalle [0,08 mm ; 0,30 mm], de préférence dans l'intervalle [0,10 mm ; 0,20 mm].

11. Prothèse implantable (16) selon l'une quelconque des revendications 1 à 10, **caractérisée en ce que** les ouvertures (18) de la structure de base (17) ont une forme allongée, ovoïde ou elliptique dont la longueur du grand axe (20) est comprise entre deux et quatre fois la longueur du petit axe (21).

12. Prothèse (16) selon la revendication 11, **caractérisée en ce que** les longueurs dudit grand axe (20) et dudit petit axe (21) sont comprises dans l'intervalle [1 mm ; 5 mm].

13. Procédé de fabrication d'une prothèse implantable (16) selon l'une quelconque des revendications 1 à 12, **caractérisé en ce que** ledit procédé comprend :
a. une étape de tricotage d'une structure de base plane (17) à mailles jetées, ayant des première et seconde faces opposées, à partir d'un fil monofilamentaire en polypropylène et d'un fil monofilamentaire en acide polylactique, ladite structure ayant des ouvertures dont au moins une dimension est supérieure ou égale à 1 mm, la structure de base (17) tricotée est tricotée à l'aide d'une première barre à passettes supportant au moins un fil monofilamentaire en polypropylène et un fil monofilamentaire en acide polylactique formant un premier fil composite (10,25), et d'une deuxième barre à passettes supportant un fil monofilamentaire en acide polylactique;
b. une étape de thermoformage de ladite structure de base plane au cours de laquelle au moins une partie de ladite structure est soumise à une déformation mécanique sous l'application d'une température déterminée et pendant une période déterminée pour conférer une forme convexe à ladite partie vue selon ladite première face et correspondant à une forme anatomique déterminée.

14. Procédé de fabrication selon la revendication 13, **caractérisé en ce qu'**il comprend une étape de sélection dudit monofilament en PLA, préliminaire à l'étape a), consistant à sélectionner un monofilament ayant une Longueur Thermoformée des Spires (L.T.S) comprise dans l'intervalle [3 mm ; 7 mm], et de préférence une Longueur Résiduelle de Spires (L.R.S.) comprise dans l'intervalle [15 mm ; 42 mm].

15. Procédé de fabrication selon l'une ou l'autre des revendications 13 et 14, caractérisé en ce la deuxième barre à passettes supporte ledit fil monofilamentaire en acide polylactique et un fil monofilamentaire en polypropylène formant un second fil composite (11,26).

## Patentansprüche

1. Implantierbare Prothese (16) zur Reparatur der Bauchwand im Inguinalbereich, insbesondere für die Total extraperitoneale Chirurgie (TEP) und/oder die Transabdominale präperitoneale Chirurgie (TAPP), umfassend eine gewirkte Basisstruktur (17), die mindestens einen Monofilamentfaden aus Polyprophylen umfasst und eine erste (17a) und zweite (17b) gegenüberliegende Seite sowie Öffnungen (18) mit mindestens einer Größe von über oder gleich 1 mm umfasst und, gesehen von der erste Seite (17a), mindestens einen konvexen Teil (19) mit einer bestimmten anatomischen Form, **dadurch gekennzeichnet, dass** die Basisstruktur (17) mindestens einen Monofilamentfaden aus Polymilchsäure (PLA) umfasst und dass die gewirkte Basisstruktur (17) mit Hilfe einer ersten Legebarre gewirkt ist, die mindestens einen Monofilamentfaden aus Polyprophylen und einen Monofilamentfaden aus Polymilchsäure trägt, die einen ersten Verbundfaden (10, 25) bilden, und einer zweiten Legebarre, die einen Monofilamentfaden aus Polymilchsäure trägt.

2. Implantierbare Prothese (16) nach Anspruch 1, **dadurch gekennzeichnet, dass** die zweite Legebarre den Monofilamentfaden aus Polymilchsäure und einen Monofilamentfaden aus Polypropylen trägt, die einen zweiten Verbundfaden (11, 26) bilden.

3. Implantierbare Prothese (16) nach dem einen oder dem anderen der Ansprüche 1 und 2, **dadurch gekennzeichnet, dass** der mindestens eine Monofilamentfaden aus Polymilchsäure eine Thermisch geformte Windungslänge (LTS) hat, die im Intervall [3 mm; 7 mm] liegt.

4. Implantierbare Prothese (16) nach einem der Ansprüche 1 bis 3, **dadurch gekennzeichnet, dass** der mindestens eine Monofilamentfaden aus Polymilchsäure eine Windungsrestlänge (LRS) hat, die im Intervall [15 mm; 42 mm] liegt.

5. Implantierbare Prothese (16) nach einem der Ansprüche 1 bis 4, **dadurch gekennzeichnet, dass** der mindestens eine Monofilamentfaden aus Polymilchsäure höchstens 10 Gew.-% seines Gewichts Einheiten der Form D, vorzugsweise höchstens 5 Gew.-% seines Gewichts Einheiten der Form D, umfasst.

6. Implantierbare Prothese (16) nach einem der Ansprüche 1 bis 5, **dadurch gekennzeichnet, dass** die gewirkte Basisstruktur (17) eine Kettwirkstruktur ist und dass der mindestens eine Monofilamentfaden aus Polyprophylen und der mindestens eine Monofilamentfaden aus Polymilchsäure Wirkmaschen bilden, die sich über mindestens drei Maschenstäbchen (12, 13, 14) erstrecken und jeweils bei einer geschlossenen Masche bis zu jeweils allen vier geschlossenen Maschen eine offene Masche (15) bilden.

7. Implantierbare Prothese (16) nach einem der Ansprüche 1 bis 6, **dadurch gekennzeichnet, dass** die Nadeln auf der oder der ersten und/oder zweiten Barre mindestens ein voll mindestens ein leer eingefädelt sind.

8. Implantierbare Prothese (16) nach Anspruch 1, **dadurch gekennzeichnet, dass** der erste Verbundfaden (10, 25) gemäß einer Anzahl von Umdrehungen/Meter verzwirnt ist, die zwischen 1 Umdrehung/Meter und 120 Umdrehungen/Meter liegt.

9. Implantierbare Prothese (16) nach Anspruch 2, **dadurch gekennzeichnet, dass** der zweite Verbundfaden (11, 26) gemäß einer Anzahl von Umdrehungen/Meter verzwirnt ist, die zwischen 1 Umdrehung/Meter und 120 Umdrehungen/Meter liegt.

10. Implantierbare Prothese (16) nach einem der Ansprüche 1 bis 9, **dadurch gekennzeichnet, dass** der mindestens eine Monofilamentfaden aus Polyprophylen und/oder der mindestens eine Monofilamentfaden aus Polymilchsäure einen Durchmesser hat oder haben, der im Intervall [0,08 mm; 0,30 mm], vorzugsweise im Intervall [0,10 mm; 0,20 mm], liegt.

11. Implantierbare Prothese (16) nach einem der Ansprüche 1 bis 10, **dadurch gekennzeichnet, dass** die Öffnungen (18) der Basisstruktur (17) eine längliche, eiförmige oder elliptische Form haben, deren Länge der großen Achse (20) zwischen dem Doppelten und dem Vierfahren der Länge der kleinen Achse (21) liegt.

12. Prothese (16) nach Anspruch 11, **dadurch gekennzeichnet, dass** die Längen der großen Achse (20) und der kleinen Achse (21) im Intervall [1 mm; 5 mm] liegen.

13. Verfahren zur Herstellung einer implantierbaren Prothese (16) nach einem der Ansprüche 1 bis 12, **dadurch gekennzeichnet, dass** das Verfahren umfasst:
a. einen Schritt des Wirkens einer ebenen Kettwirk-Basisstruktur (17) mit einer ersten und zweiten gegenüberliegenden Seite aus einem Monofilamentfaden aus Polyprophylen und einem Monofilamentfaden aus Polymilchsäure, wobei die Struktur Öffnungen hat, deren mindestens eine Größe größer oder gleich 1 mm ist, wobei die gewirkte Basistruktur (17) mit Hilfe einer ersten Legebarre gewirkt ist, die mindestens einen Monofilamentfaden aus Polyprophylen und einen Monofilamentfaden aus Polymilchsäure trägt, die einen ersten Verbundfaden (10, 25) bilden, und einer zweiten Legebarre, die einen Monofilamentfaden aus Polymilchsäure trägt;
b. einen thermischen Formungsschritt der ebenen Basisstruktur, bei dem mindestens ein Teil der Struktur einer mechanischen Verformung unter Anwendung einer bestimmten Temperatur und während eines bestimmten Zeitraums unterzogen wird, um dem Teil, gesehen von der erste Seite und entsprechend einer bestimmten anatomischen Form, eine konvexe Form zu verleihen.

14. Herstellungsverfahren nach Anspruch 13, **dadurch gekennzeichnet, dass** es einen Auswahlschritt des Monofilaments aus PLA vor dem Schritt a) umfasst, der darin besteht, ein Monofilament mit einer thermisch geformten Windungslänge (LTS) auszuwählen, die im Intervall [3 mm; 7 mm] liegt und vorzugsweise einer Windungsrestlänge (LRS), die im Intervall [15 mm; 42 mm] liegt.

15. Herstellungsverfahren nach dem einen oder anderen der Ansprüche 13 und 14, **dadurch gekennzeichnet, dass** die zweite Legebarre den Monofilamentfaden aus Polymilchsäure und einen Monofilamentfaden aus Polypropylen trägt, die einen zweiten Verbundfaden (11, 26) bilden.

## Claims

1. An implantable prosthesis (16) intended to repair the abdominal wall in the inguinal region, in particular for Totally Extra-Peritoneal (T.E.P) surgery and/or Trans-Abdominal Pre-Peritoneal (T.A.P.P) surgery, comprising a knitted base structure (17) comprising at least one polypropylene monofilament yarn and having first (17a) and second (17b) opposite faces as well as openings (18) having at least one dimension greater than or equal to 1 mm, and, seen according to said first face (17a), at least one convex part (19) having a determined anatomical shape, **characterized in that** the base structure (17) comprises at least one polylactic acid (PLA) monofilament yarn and **in that** the knitted base structure (17) is knitted using a first guide bar supporting at least one polypropylene monofilament yarn and one polylactic acid monofilament yarn forming a first composite yarn (10,25), and a second guide bar supporting a polylactic acid monofilament yarn.

2. The implantable prosthesis (16) according to claim 1, **characterized in that** the second guide bar supports the polylactic acid monofilament yarn and a polypropylene monofilament yarn forming a second composite yarn (11,26).

3. The implantable prosthesis (16) according to either of claims 1 and 2, **characterized in that** said at least one polylactic acid monofilament yarn has a Thermoformed Length of Turns (L.T.S) comprised in the interval [3 mm; 7mm].

4. The implantable prosthesis (16) according to any one of claims 1 to 3, **characterized in that** said at least one polylactic acid monofilament yarn has a Residual Length of Turns (L.R.S.) comprised in the interval [15 mm; 42mm].

5. The implantable prosthesis (16) according to any one of claims 1 to 4, **characterized in that** said at least one polylactic acid monofilament yarn comprises at most 10% by weight of its weight of D-shaped units, preferably not more than 5% by weight of its weight of D-shaped units.

6. The implantable prosthesis (16) according to any one of claims 1 to 5, **characterized in that** the knitted base structure (17) is knitted with warp stitches, and **in that** said at least one polypropylene monofilament yarn and said at least one polylactic acid monofilament yarn form knitted stitches extending over at least three columns of stitches (12,13,14) and form an open stitch (15) every one closed stitch to every four closed stitches.

7. The implantable prosthesis (16) according to any one of claims 1 to 6, **characterized in that** the needles on the first and/or second bar(s) is/are yarned at least one full at least one empty.

8. The implantable prosthesis (16) according to claim 1, **characterized in that** the first composite yarn (10,25) is twisted according to a number of turns/meter comprised between 1 turn/meter and 120 turns/meter.

9. The implantable prosthesis (16) according to claim 2, **characterized in that** the second composite yarn (11,26) is twisted according to a number of turns/meter comprised between 1 turn/meter and 120 turns/meter.

10. The implantable prosthesis (16) according to any one of claims 1 to 9, **characterized in that** said at least one polypropylene monofilament yarn and/or said at least one polylactic acid monofilament yarn has or have a diameter comprised in the interval [0.08 mm; 0.30 mm], preferably in the interval [0.10 mm; 0.20mm].

11. The implantable prosthesis (16) according to any one of claims 1 to 10, **characterized in that** the openings (18) of the base structure (17) have an elongated, ovoid or elliptical shape, the length of the major axis of which (20) is comprised between two and four times the length of the minor axis (21).

12. The prosthesis (16) according to claim 11, **characterized in that** the lengths of said major axis (20) and of said minor axis (21) are comprised in the interval [1 mm; 5mm].

13. A method for manufacturing an implantable prosthesis (16) according to any one of claims 1 to 12, **characterized in that** said method comprises:
a. a step of knitting a planar base structure (17) with warp stitches, having first and second opposite faces, from a polypropylene monofilament yarn and a polylactic acid monofilament yarn, said structure having openings of which at least one dimension is greater than or equal to 1 mm, the knitted base structure (17) is knitted using a first guide bar supporting at least one polypropylene monofilament yarn and one polylactic acid monofilament yarn forming a first composite yarn (10,25), and a second guide bar supporting a polylactic acid monofilament yarn;
b. a step of thermoforming said planar base structure during which at least a part of said structure is subjected to mechanical deformation under the application of a determined temperature and for a determined period to confer a convex shape to said part seen according to said first face and corresponding to a determined anatomical shape.

14. The manufacturing method according to claim 13, **characterized in that** it comprises a step of selecting said PLA monofilament, prior to step a), consisting in selecting a monofilament having a Thermoformed Length of Turns (L.T.S) comprised in the interval [3 mm; 7 mm], and preferably a Residual Length of Turns (R.S.L.) comprised in the interval [15 mm; 42mm].

15. The manufacturing method according to either of claims 13 and 14, **characterized in that** the second guide bar supports said polylactic acid monofilament yarn and a polypropylene monofilament yarn forming a second composite yarn (11,26).
